# EUROPEAN PATENT SPECIFICATION

(11) **EP 0 820 565 B1**
(45) Date of publication and mention of the grant of the patent: **19.01.2000**
(21) Application number: 96909272.5
(22) Date of filing: 04.04.1996
(51) Int. Cl.: F16K 31/60, G05G 1/08, A61F 5/44

(54) **A TAP**
HAHN
ROBINET

(30) Priority: 11.04.1995 GB 9507507
(43) Date of publication of application: 28.01.1998
(73) Proprietor: Simpla Plastics Limited, Oldham Lancastershire OL1 3HS (GB)
(72) Inventor: KARLSEN, Sven, Belton Doncaster DN9 1TG (GB)
(74) Representative: Johnson, Terence Leslie
(86) International application number: GB9600865
(87) International publication number: WO9632602

(56) References cited:
- EP-A- 0 418 579
- EP-A- 0 580 384
- US-A- 3 922 790
- US-A- 4 844 415

## Description

The invention relates to a tap, and in particular to the kind of tap adapted for use in drainage of bodily fluids. The invention further relates to a urine drainage bag including such a tap and apparatus for delivery or drainage of fluids including such a tap.

Taps are used in a wide variety of applications, and in some of them, a problem which has been experienced with taps of known design is that whilst it is necessary to provide some form of handle which is easy for the user to grip and operate, such handles generally protrude some distance from the body of the tap itself, making the tap bulky and awkward in storage and use. One area where this problem is particularly acute is that of urine drainage apparatus, where a patient who is catheterised has a drainage bag for collection of urine, which is provided with a tap to enable it to be easily emptied. The tap is customarily connected to the bag in a length of tubing close to the person's body and this can give rise to problems in the case where the tap has a projecting lever arm which is moveable between a closed position generally parallel to the length of tubing and an open position generally at right angles thereto. The projecting lever arm may press into the person's body and may be inadvertently snagged by being caught on bed clothing, sleeping apparel, or on the clothing of people such as nurses passing by or standing near a patient using urine drainage apparatus.

A number of solutions have been suggested to this problem, for example taps including devices whereby the lever arm remains closely adjacent the tubing in both the open and closed positions. Such a tap is shown in EP-A-0580384, while another tap, shown in US-A-48444115 which relies on a spigot and socket kind of arrangement, always has a projection in the form of the spigot to cause snagging. Other taps for tanks or tape measures are shown in EP-A-0418579 and US-A-3922790. None of these devices, however, provides a tap for urine drainage which is completely free from protuberances which may become snagged. They also involve relatively complicated mechanisms which are expensive to manufacture and also difficult to clean.

This invention seeks to mitigate problems such as these.

According to a first aspect of the invention there is provided a tap for urine drainage, comprising a body, a control member within the body moveable between open and closed positions, and operating means for operating the control member, characterised by the control member having a recess disposed entirely within the perimeter thereof, and by the operating means being moveable between an operating position and a stowed position in which latter position the operating means is received in the recess and lies within the perimeter of the control member.

Using the invention it is possible to eliminate the need for complex mechanisms to allow a handle of the tap to remain flush with the tubing in both the open and closed positions, and further provides a tap which in both open and closed positions can have a virtually protuberance-free outer surface, thereby substantially obviating inadvertent operation or snagging.

In particular, the control member may comprise a recess, into which the part is retractable partially or fully. This provides a particularly smooth and snag-free surface.lt is particularly preferred that the part is retractable to be substantially flush with a surface of the control member.

The part may be hingedly attached to a part of the control member for movement between the operating and stowed positions. This is particularly convenient.

The operating means, the hinge and the control member may be integrally formed. This provides for ease of construction.

An alternative yet equally effective arrangement is to have the operating means moveable between the operating and stowed positions by sliding, relative to the control member.

The operating means may, again, be removed completely from the control member, and inserted into an aperture thereof for operation.

It is preferred that the operating means is retained in the stowed position by retaining means. This prevents the operating means from inadvertently being moved from its stowed position. The retaining means may comprise mutually co-operable parts of the operating means and the control member.

The tap may be provided with biassing means to bias the operating means towards the unretracted or operative position when the retaining means is released. This prevents the handle from falling back into the stowed position when the tap is in use.

For ease of use, it is preferred that the operating part of the operating means may comprise a substantially planar tab which can easily be gripped by a user.

According to a second aspect of the invention there is provided a tap as hereinbefore defined, adapted for use in drainage of bodily fluids.

According to a third aspect of the invention there is provided a urine drainage bag, including a tap as hereinbefore defined.

According to a fourth aspect of the invention there is provided apparatus for delivery or drainage of fluids, including a tap as hereinbefore defined.

Taps embodying the invention are hereinafter described, by way of example, with reference to the accompanying drawings.
Fig. 1 is a front view of an embodiment of tap according to the invention in a closed, stowed or inoperative position;
Fig. 2 is a front view of the tap of Fig. 1 in the open or operative position;
Fig. 3 is a front perspective view of a control member for use in the embodiment of tap shown in Figs. 1 and 2, in the unretracted or operative position;
Fig. 4 is a front view of a further embodiment of tap according to the invention; and
Fig. 5 is an underplan view of the tap shown in Figs. 1 and 2.

Referring to the drawings in which like parts are indicated by like numerals, there is shown a tap 1 comprising a body or barrel 2, a control member 3 within the body 2, moveable between open and closed positions, and stowable operating means 4 for operating the control member 3, the arrangement being such that in the stowed position, the operating means 4 lies substantially within the perimeter of the control member 3. This therefor obviates inadvertent operation or snagging.

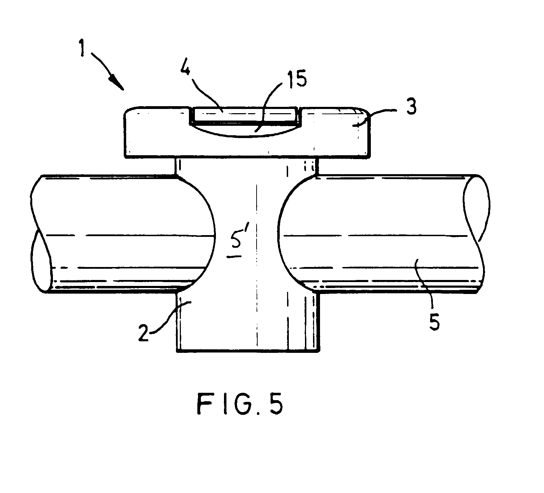

The tap 1 comprises a length of tube 5 with a transverse cylindrical barrel 5' (shown most clearly in Fig. 5) in which is received in rotatably slidable relation in a cylindrical barrel 6 of the control member 3 the barrel 6 having a through, diametrically extending, bore 7 of substantially the same diameter as the tube 5 for passage of fluid when the tap is in the open position for fluid flow along the tube 5.

At one end, the control member 3, which has a close fit in the barrel 5 but can turn with respect thereto as described before, carries the operating means 4, which in the embodiment shown takes the form of a flap or tab 9 which is attached to the control member 3 by two integral hinges 8 and which is generally planar, and has an end part opposite the hinges 8 for gripping by a user. The flap 9 is provided with biassing means which in the illustrated embodiment is in the form of an over-centre integral flat spring member 10 which serves to help keep the flap in the unretracted or retracted positions. Retaining means for the flap 9 is provided in the form of a spigot and socket arrangement, the flap being provided with the spigot 11 on its inner (in use) surface which cooperates as a push fit with a socket 12 located on a corresponding part of the control member 3.

In the illustrated embodiment, in the stowed or retracted position the flap 9 lies flush with the front (outer as viewed) surface of the control member 3. This is achieved by forming a recess 13 in the surface 14 of the control member 3 having a depth of substantially the thickness of the flap 9. In order to make the flap more easily accessible, a part 15 of the control member 3 located below the free end of the flap in the retracted position is cut away so that the end of the flap forms a slight overhang. This provides a grip, which makes the flap more easily openable.

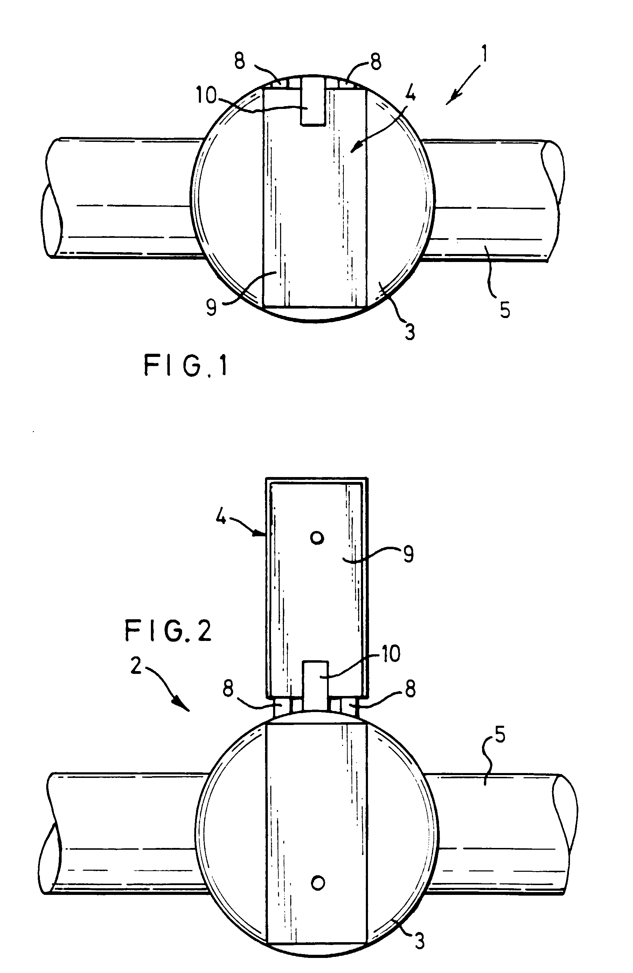

In use, in the retracted position illustrated in Fig. 1 the operating means 4 is retained by the retaining means in its recess 13, flush with the surface of the control member 3 and within the perimeter or surface area of the end thereof. When it is desired to operate the tap from the open to the closed position, or vice versa, the stowed operating means 4 (Fig. 1) is gripped via the part 15 and pivoted about 180° by means of the hinges 8 to the open or unstowed position illustrated in Fig. 2. The position of the tap 1 may then be changed by rotating the operating means, and thereby the control means 3 to the desired position. When the bore 7 is either aligned with the tube 5 for flow, or is at 90° thereto to cut off flow and close the tap, the desired open or closed position of the tap is achieved, the operating means 4 is then returned to the recess 13 where it is held securely in place by the retaining means.

The tap may be manufactured from any suitable material, such as a plastics material. In particular, the control member 3 may be conveniently manufactured in two parts, namely the end part bearing the operating means 4 and the barrel part bearing the bore 7. The two parts can be secured together as by snap engagement.

It will be appreciated that a large number of variations are possible. For example, the shape of the flap can be varied to optimise its function as an operating means 4 in the unretracted position. In particular, a variety of different shapes of flap 9 may be provided.

Although the control member 3 shown in the embodiment is substantially cylindrical, it could be a variety of different shapes, such as for example oval, as long as the basic anti-snag function is not impaired (the barrel 6 remaining cylindrical).

In the embodiment the operating means 4 is received in the recess 13, however it will be appreciated that a relatively smooth and snag-free arrangement is possible, even without providing any recess.

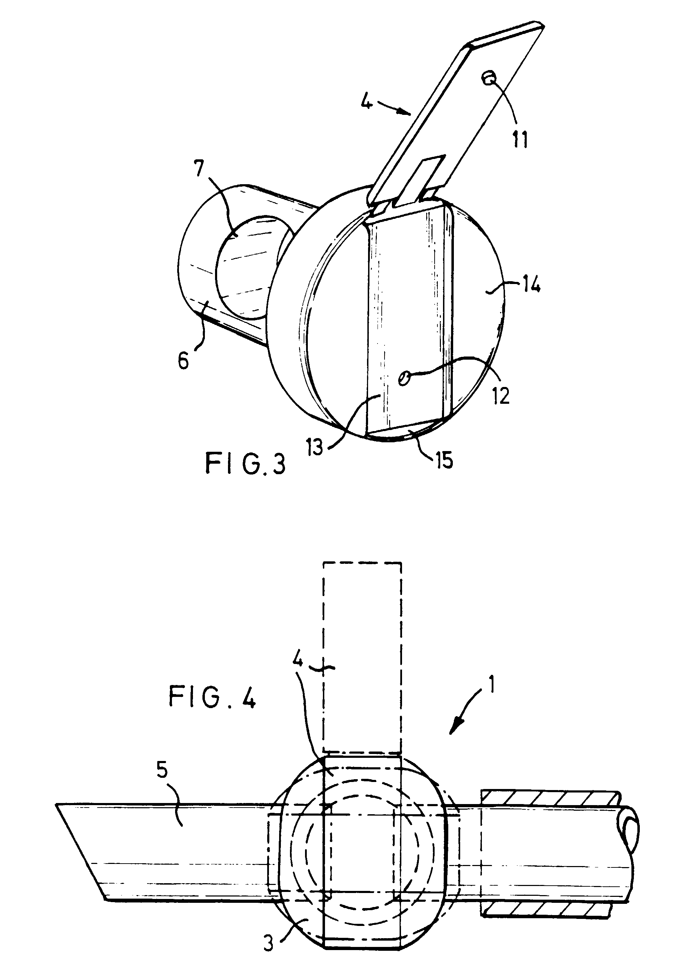

Thus it is envisaged that the operating means 4 could be moveable from the stowed or retracted to the unstowed or unretracted position in ways other than by hinging. One particularly effective arrangement is to have the flap 9 and recess 13 in slidable engagement, the flap being slidable sideways out of the recess until a stop means is reached. This is illustrated in Fig. 4, the operating or non-stowed position of the tab 9 being shown in chain-dash lines.

In another embodiment, the flap 9 could be entirely separate from, but stowed inside the control member 3. When it is desired to operate the tap 1 the tab or flap 9 would be removed and inserted in for example, an appropriate recess in the control member 3 thereby providing a handle for operation of the tap 1. The flap 9 forms an operating handle in all embodiments.

As can be seen from the foregoing description, taps embodying the invention and described with reference to the accompanying drawings are relatively easy to manufacture suitably by moulding in plastic, and are yet simple in configuration. They are also easy to operate in practice without the drawback of "snagging".

## Claims

1. A tap for urine drainage, comprising a body, a control member within the body moveable between open and closed positions, and operating means for operating the control member, characterised by the control member (3) having a recess (13) disposed entirely within the perimeter thereof, and by the operating means (9) being moveable between an operating position and a stowed position in which latter position the operating means (9) is received in the recess (13) and lies within the perimeter of the control member (3).

2. A tap according to Claim 1, characterised by the operating means (9) being stowable in the recess (13) so as to lie substantially flush with a surface (14) of the control member (3).

3. A tap according to either preceding claim, characterised by the operating means (9) being attached by hinged means (8) to a part of the control member (3) for movement between the operating and stowed positions.

4. A tap according to Claim 3, characterised by the operating means (9), the hinge means (8) and the control member (3) being integrally formed.

5. A tap according to either of Claims 1 or 2, characterised by the operating means (9) being moveable between the operating and stowed positions by sliding relative to the control member.

6. A tap according to either of Claims 1 or 2, characterised by the operating means (9) being removable completely from the control member (3), and inserted into a recess thereof for operation.

7. A tap according to any preceding claim, characterised by the operating means (9) being retained in the stowed position by retaining means (11, 12).

8. A tap according to Claim 7, characterised by the retaining means (11, 12) comprising mutually co-operable respective parts (11, 12) of the operating means (9) and the control member (3).

9. A tap according to any of Claims 1 to 4, characterised by biassing means (10) adapted to bias the operating means (9) towards the operating position.

10. A tap according to any preceding claim, characterised by the operating means (9) comprising a substantially planar tab.

11. A tap according to any preceding claim, adapted for use in drainage of bodily fluids.

12. A urine drainage bag, including a tap according to any of Claims 1 to 11.

13. Apparatus for delivery or drainage of fluids, including a tap as claimed in any of Claims 1 to 11.

## Patentansprüche

1. Hahn für Urinabfluß, bestehend aus einem Körper, einem Steuermittel in dem Körper, das zwischen geöffneten und geschlossenen Stellungen beweglich ist, und einem Betätigungsmittel zur Betätigung des Steuermittels, dadurch gekennzeichnet, daß das Steuermittel (3) eine Vertiefung (13) aufweist, die vollständig innerhalb seines Umfangs angeordnet ist, und daß das Betätigungsmittel (9) zwischen einer Betätigungsstellung und einer Unterbringungsstellung beweglich ist, wobei das Betätigungsmittel (9) in der letztgenannten Stellung in der Vertiefung (13) aufgenommen wird und innerhalb des Umfangs des Steuermittels (3) liegt.

2. Hahn nach Anspruch 1, dadurch gekennzeichnet, daß das Betätigungsmittel (9) in der Vertiefung (13) unterbringbar ist, so daß es mit einer Oberfläche (14) des Steuermittels (3) im wesentlichen fluchtend liegt.

3. Hahn nach einem der vorstehenden Ansprüche, dadurch gekennzeichnet, daß das Betätigungsmittel (9) mit einem schwenkbaren Mittel (8) an einen Teil des Steuermittels (3) für Bewegung zwischen der Betätigungsstellung und der Unterbringungsstellung angebracht ist.

4. Hahn nach Anspruch 3, dadurch gekennzeichnet, daß das Betätigungsmittel (9), das schwenkbare Mittel (8) und das Steuermittel (3) einstückig miteinander sind.

5. Hahn nach Anspruch 1 oder 2, dadurch gekennzeichnet, daß das Betätigungsmittel (9) durch Verschieben relativ zum Steuermittel zwischen der Betätigungsstellung und der Unterbringungsstellung beweglich ist.

6. Hahn nach Anspruch 1 oder 2, dadurch gekennzeichnet, daß das Betätigungsmittel (9) vollständig vom Steuermittel (3) entfernt werden kann und für Betätigung in eine Vertiefung davon eingesteckt wird.

7. Hahn nach einem der vorstehenden Ansprüche, dadurch gekennzeichnet, daß das Betätigungsmittel (9) in der Unterbringungsstellung durch Haltemittel (11, 12) gehalten wird.

8. Hahn nach Anspruch 7, dadurch gekennzeichnet, daß die Haltemittel (11, 12) aus gegenseitig zusammenwirkenden Teilen (11, 12) des Betätigungsmittels (9) bzw. des Steuermittels (3) bestehen.

9. Hahn nach einem der Ansprüche 1 bis 4, gekennzeichnet durch ein Vorbelastungsmittel (10) zum Vorbelasten des Betätigungsmittels (9) auf die Betätigungsstellung zu.

10. Hahn nach einem der vorstehenden Ansprüche, dadurch gekennzeichnet, daß das Betätigungsmittel (9) eine im wesentlichen ebene Zunge aufweist.

11. Hahn nach einem der vorstehenden Ansprüche, der zur Verwendung beim Abfluß von Körperflüssigkeiten angewandt werden kann.

12. Urinabflußbeutel, der einen Hahn nach einem der Ansprüche 1 bis 11 enthält.

13. Vorrichtung zum Zuführen oder zum Abfließen von Flüssigkeiten, die einen Hahn nach einem der Ansprüche 1 bis 11 enthält.

## Revendications

1. Drain pour le drainage de l'urine, comprenant un corps, un élément de commande à l'intérieur du corps qui peut être déplacé entre des positions ouverte et fermée, et un moyen d'activation pour activer l'élément de commande, caractérisé en ce que l'élément de commande (3) comporte un évidement (13) disposé en totalité à l'intérieur de son périmètre et en ce que le moyen d'activation (9) peut être déplacé entre une position d'activation et une position de rangement, seconde position dans laquelle le moyen d'activation (9) est reçu dans l'évidement (13) et s'étend à l'intérieur du périmètre de l'élément de commande (3).

2. Drain selon la revendication 1, caractérisé en ce que le moyen d'activation (9) peut être rangé dans l'évidement (13) de manière à s'étendre de façon à affleurer sensiblement une surface (14) de l'élément de commande (3).

3. Drain selon l'une quelconque des revendications précédentes, caractérisé en ce que le moyen d'activation (9) est fixé par un moyen de charnière (8) à une partie de l'élément de commande (3) en vue d'un déplacement entre les positions d'activation et de rangement.

4. Drain selon la revendication 3, caractérisé en ce que le moyen d'activation (9). le moyen de charnière (8) et l'élément de commande (3) sont formés d'un seul tenant.

5. Drain selon l'une quelconque des revendications 1 et 2. caractérisé en ce que le moyen d'activation (9) peut être déplacé entre les positions d'activation et de rangement en réalisant un coulissement par rapport à l'élément de commande.

6. Drain selon l'une quelconque des revendications 1 et 2, caractérisé en ce que le moyen d'activation (9) peut être ôté complètement de l'élément de commande (3) et inséré dans un évidement de celui-ci pour son activation.

7. Drain selon l'une quelconque des revendications précédentes, caractérisé en ce que le moyen d'activation (9) est retenu dans la position de rangement par un moyen de retenue (11, 12).

8. Drain selon la revendication 7, caractérisé en ce que le moyen de retenue (11, 12) comprend des parties respectives pouvant coopérer de façon mutuelle (11, 12) du moyen d'activation (9) et de l'élément de commande (3).

9. Drain selon l'une quelconque des revendications 1 à 4, caractérisé en ce qu'un moyen de poussée (10) est adapté pour pousser le moyen d'activation (9) en direction de la position d'activation.

10. Drain selon l'une quelconque des revendications précédentes. caractérisé en ce que le moyen d'activation (9) comprend une patte sensiblement plane.

11. Drain selon l'une quelconque des revendications précédentes, adapté pour une utilisation lors du drainage de fluides du corps.

12. Poche de drainage de l'urine incluant un drain selon l'une quelconque des revendications 1 à 11.

13. Appareil pour délivrer ou drainer des fluides, incluant un drain selon l'une quelconque des revendications 1 à 11.
